# EUROPÄISCHE PATENTSCHRIFT

(11) **EP 2 341 835 B1**
(45) Veröffentlichungstag und Bekanntmachung des Hinweises auf die Patenterteilung: **12.02.2014**
(21) Anmeldenummer: 09769005.1
(22) Anmeldetag: 25.06.2009
(51) Int. Cl.: A61B 10/02

(54) **ANORDNUNG ZUR ZERSTÖRUNG VON TUMORZELLEN UND TUMORGEWEBE**
ARRANGEMENT FOR DESTROYING TUMOUR CELLS AND TUMOUR TISSUE
DISPOSITIF POUR LA DESTRUCTION DE CELLULES TUMORALES ET DE TISSUS TUMORAUX

(30) Priorität: 25.06.2008 DE 102008030213
(43) Veröffentlichungstag der Anmeldung: 13.07.2011
(73) Patentinhaber: Oncowave Medical AG, 64295 Darmstadt (DE)
(72) Erfinder: Theuer, Axel Erich, 88486 Kirchberg (DE)
(74) Vertreter: Kilchert, Jochen
(86) Internationale Anmeldenummer: PCT/EP2009/004593
(87) Internationale Veröffentlichungsnummer: WO 2009/156156

(56) Entgegenhaltungen:
- WO-A-83/02718
- WO-A-98/07470
- WO-A-2005/065408
- WO-A-2005/074365
- WO-A-2006/018837
- DE-A1- 3 919 592
- DE-A1- 4 414 239
- US-A- 5 156 144
- US-A- 5 601 526
- US-A- 5 618 275
- US-A- 5 827 204
- US-A1- 2003 073 992

## Beschreibung

Die Erfindung betrifft eine Anordnung zur Zerstörung von Tumorzellen und Tumorgewebe gemäß Anspruch 1.

DE 100 23 457 A1 zeigt ein Ultraschallgerät zur therapeutischen Behandlung von Tumorerkrankungen. Es ist bekannt, dass Tumorzellen bei einer Anregung durch Ultraschallwellen gegenüber gesunden Zellen ein verändertes Schwingungsverhalten aufweisen. Schwingungsrelevante Zellparameter sind die Steifigkeit und die Beschaffenheit des Zytoskeletts, die Viskosität des Zytoplasmas, der Plasmamembran und der Kernflüssigkeit, die Kern/Plasma-Relation, der osmotische Druck, die Steifigkeit und Beschaffenheit der extrazellulären Matrix, die Viskosität der extrazellulären Flüssigkeit und die Geschwindigkeit des zellulären Aggregationsprozesses. Maligne Zellen weisen gesunden Zellen gegenüber ein verändertes Zytoskelett auf, so zeigen invasive Tumorzellen einen Umbau ihrer Intermediärfilamente. Die Kern/Plasma-Relation ist zu Gunsten des Zellkerns verschoben. Der Zellkern maligner Zellen ist oft in seiner Größe verändert und hat eine erhöhte Chromatindichte.

Verschiedene Therapiemethoden machen sich dieses veränderte Schwingungsverhalten zunutze. So lassen sich Tumorzellen mit einer ihnen eigenen Resonanzfrequenz anregen und durch eine kontrollierte Beschallung mit Ultraschall zerstören.

Die Resonanzfrequenzen der einzelnen Tumorzellen sind allerdings nicht einheitlich. Des Weiteren sind die Unterschiede im Schwingungsverhalten von gesunden Zellen und von erkrankten Zellen nicht regelmäßig so groß, dass eine Schädigung der gesunden Zellen bei der üblichen Behandlung mittels Ultraschall sicher vermieden oder zumindest verringert werden kann. Zwar kann man die Bandbreite des verwendeten Ultraschalls und dessen Leistung reduzieren. Jedoch bedingt diese Reduktion, dass zahlreiche Tumorzellen von der Behandlung nicht erfasst werden, da die Tumorzellen heterogen sind.

Ausgehend von diesem Stand der Technik ist es Aufgabe der vorliegenden Erfindung, eine Anordnung zur verbesserten Behandlung von Tumorerkrankungen bereitzustellen.

Diese Aufgabe wird erfindungsgemäß durch den Gegenstand des Anspruchs 1 gelöst.

Die Erfindung betrifft eine Anordnung zur Zerstörung von Tumorzellen, die Folgendes umfasst: eine therapeutische Vorrichtung mit wenigstens einem Hochfrequenz-Ultraschallgenerator zur Erzeugung einer thermisch wirksamen, hochfrequenten Schwingung mit mindestens 0,25 MHz, mehreren ersten Niederfrequenz-Ultraschallgeneratoren zur Erzeugung einer therapeutisch wirksamen, niederfrequenten Schwingung im angegebenen Frequenzbereich, wobei die ersten Niederfrequenz-Ultraschallgeneratoren jeweils eine unterschiedliche Frequenz erzeugen bzw. dazu angepasst sind, und einer Steuerung, die mit dem Hochfrequenz-Ultraschallgenerator und den ersten Niederfrequenz-Ultraschallgeneratoren verbunden ist derart, dass die Tumorzellen mit einer hochfrequenten, auf die Tumorzellen thermisch wirkenden Schwingung und mit einer niederfrequenten Schwingung beaufschlagbar sind. Zusätzlich zu der vorstehend genannten Vorrichtung umfasst die Anordnung eine Biopsievorrichtung mit mehreren Einzelaufnahmen für Gewebeproben und mehreren zweiten Niederfrequenz-Ultraschallgeneratoren, die mit den Einzelaufnahmen zur Beaufschlagung der Gewebeproben mit Schwingungen wirkverbunden sind. Dabei sind durch jeweils einen ersten und zweiten Niederfrequenz-Ultraschallgenerator Schwingungen mit derselben Frequenz erzeugbar.

Die erfindungsgemäße Anordnung beruht auf dem Prinzip, die mit der Biopsievorrichtung zum Testen von Gewebeproben erzeugbaren bzw. verwendeten Schwingungsfrequenzen mit den zur Behandlung des Tumors durch die Vorrichtung verwendeten Schwingungsfrequenzen zu korrelieren. Dadurch werden die Behandlungserfolge signifikant verbessert, da eine tumorspezifische, an das jeweilige Stadium des Tumors angepasste wirksame Frequenz vorab bestimmt und mit der Vorrichtung verwendet werden kann.

Insbesondere wird die Aufgabe durch eine Vorrichtung zur Zerstörung von Tumorzellen gelöst, wobei die Vorrichtung wenigstens einen Hochfrequenz-Ultraschallgenerator zur Erzeugung einer thermisch wirksamen hochfrequenten Schwingung, wenigstens einen Niederfrequenz-Ultraschallgenerator zur Erzeugung einer therapeutisch wirksamen, niederfrequenten Schwingung und eine Steuerung umfasst, die mit dem Hochfrequenz-Ultraschallgenerator und dem Niederfrequenz-Ultraschallgenerator derart verbunden ist, dass die Tumorzellen mit einer hochfrequenten, auf die Tumorzellen thermisch wirkende, Schwingung und mit einer niederfrequenten Schwingung beaufschlagbar sind, wobei die niederfrequente Schwingung auf eine die Tumorzelle zerstörende Frequenz durch die Steuerung einstellbar ist.

Die Erfindung beruht also darauf, zwei unterschiedliche Ultraschallgeneratoren einzusetzen, und zwar einen Niederfrequenz-Ultraschallgenerator (NF-Ultraschallgenerator) und einen Hochfrequenz-Ultraschallgenerator (HF-Ultraschallgenerator). Die Zerstörung der Tumorzellen und mithin des Tumorgewebes mit dieser Vorrichtung erfolgt durch das Anregen der Tumorzellen mit einer niederfrequenten Schwingung, die durch den NF-Ultraschallgenerator erzeugt wird und auf die entsprechenden Tumorzellen abgestimmt ist. Durch ein vorhergehendes oder gleichzeitiges, insbesondere lokales Erwärmen der zu behandelnden Tumorzellen mittels des HF-Ultraschallgenerators werden bessere Behandlungserfolge erzielt.

Insbesondere führt die Erwärmung der Tumorzellen bzw. des Tumorgewebes durch die Hochfrequenzschwingung zu einer besseren selektiven Unterscheidbarkeit der Tumorzellen von gesunden Zellen. Durch die lokale, gezielte Erwärmung der Tumorzellen wird ein Temperaturgefälle zwischen den gesunden Zellen und den kranken Zellen eingestellt. Das Temperaturgefälle sorgt dafür, dass die Unterschiede im Schwingungsverhalten der kranken und gesunden Zellen verstärkt werden. Durch das stärker abgegrenzte Schwingungsverhalten wird das Targetproblem besser gelöst, da eine schärfere Abgrenzung des Schwingungsverhaltens der kranken Zellen dazu führt, dass die selektive Anregung der kranken Zellen durch die Beaufschlagung mit niederfrequentem Ultraschall verbessert wird. Der Vorteil der Erfindung besteht darin, dass durch den Hochfrequenz-Ultraschallgenerator die Voraussetzung dafür geschaffen wird, dass thermische Energie lokal begrenzt auf die kranken Zellen in den Körper eingetragen werden kann und zwar auf einfache und unkomplizierte Weise. Damit wird das Selektionskriterium, nämlich das Schwingungsverhalten der kranken und gesunden Zellen und somit der Behandlungserfolg signifikant verbessert.

Die Vorrichtung kann eine Halteeinrichtung umfassen, in der der Hochfrequenz-Ultraschallgenerator, insbesondere dessen Ultraschallkopf, und der Niederfrequenz-Ultraschallgenerator, insbesondere dessen Ultraschallkopf, gehalten sind. Für einen guten Erfolg der Behandlung ist eine geeignete Positionierbarkeit der Ultraschallgeneratoren bzw. deren Ultraschallköpfe insbesondere des HF-Ultraschallkopfes zweckmäßig. Diese Positionierung kann gegenüber dem zu behandelnden Gewebe mittels einer entsprechenden Halteeinrichtung erfolgen. Hierfür können die Ultraschallgeneratoren in der Halteeinrichtung in einer Gegenüberstellung gehalten werden.

Die Halteeinrichtung kann glocken- oder schüsselartig ausgebildet sein und zumindest teilweise einen Behandlungsraum begrenzen. Somit kann die Halteeinrichtung ein Medium, insbesondere ein Fluid, aufnehmen, das eine verbesserte Applikation der hochfrequenten und der niederfrequenten Schwingungen ermöglicht. Insbesondere können Reflexionen an den Grenzflächen (z.B. zwischen Luft und menschlichem Gewebe), eine ungewünschte Absorbation (beispielsweise vor dem Eintritt der Schallwellen in das zu behandelnde Gewebe) und eine Streuung beim Eintritt des Schalls in das Gewebe vermieden oder reduziert werden. Aufgrund der Ausgestaltung der Halteeinrichtung kann diese als Unterdruckkammer oder als Überdruckkammer fungieren und die Platzierung der Vorrichtung am zu behandelnden Gewebe erleichtern. Durch eine der Kammer zugeordnete Drucksteuerung kann das Kavitätsverhalten bei der Ultraschallbehandlung beeinflusst werden. Dabei werden durch Einstellung eines Überdrucks in der Kammer Kavitationsphänomene unterdrückt und durch Einstellen eines Unterdrucks werden Kavitationsphänomene unterstützt.

Die Vorrichtung kann eine Fluidpumpe umfassen, die zur Zirkulation und/oder zum Einbringen und/oder zum Abführen eines Fluids über eine Fluidleitung mit dem Behandlungsraum verbunden ist. Der Unterdruck für die Unterdruckkammer bzw. der Überdruck für die Überdruckkammer kann also durch die Fluidpumpe hergestellt werden. Des Weiteren kann über die Fluidleitung, je nach Bedarf, ein Medium bzw. Additive zum Fluid in den Behandlungsraum eingebracht oder aus diesem abgeführt werden. Diese Additive können beispielsweise Sonosensitizer umfassen.

Die Fluidpumpe kann eine Heiz- und/oder Kühleinheit zur Einstellung einer vorbestimmten Temperatur im Behandlungsraum umfassen. Durch ein externes Erwärmen oder Abkühlen des zu behandelnden Gewebes mittels des Fluids kann der Behandlungserfolg weiter erhöht werden. Insbesondere kann die selektive Anregung, die bei der Applikation des niederfrequenten Ultraschalls erzielt werden soll, verbessert werden.

Wenigstens der HF-Ultraschallgenerator kann eine Schalllinse umfassen, die die erzeugten Schwingungen fokussiert. Andere Fokussiermittel sind möglich, beispielsweise ein HF-Ultraschallkopf mit Schwingungsarrays, also einer Vielzahl ansteuerbare Schwingungselemente. Der fokussierende HF-Ultraschallgenerator hat den Vorteil, dass ein räumlich begrenztes Tumorgewebe erwärmt werden kann.

Die Steuerung ist derart angepasst, dass mit dem HF-Ultraschallgenerator eine hochfrequente Schwingung mit wenigstens 0,25 MHz (Megahertz), insbesondere mit wenigstens 0,5 MHz, insbesondere mit wenigstens 0,8 MHz, insbesondere mit wenigstens 1 MHz, erzeugbar ist. Der HF-Ultraschallgenerator ist dementsprechend für die besagten Frequenzen optimiert.

Die Steuerung ist derart angepasst, dass mit dem NF-Ultraschallgenerator eine Schwingung erzeugbar ist, die im Bereich von einem kHz (Kilohertz) bis 200 kHz, insbesondere 1 kHz bis 150 kHz, insbesondere 5 kHz bis 150 kHz, insbesondere 10 kHz bis 150 kHz, insbesondere 16 kHz bis 150 kHz, insbesondere 20 kHz bis 150 kHz, insbesondere 20 kHz bis 80 kHz, insbesondere 20 kHz bis 40 kHz, variierbar ist. Die niederfrequente Schwingung sollte derart einstellbar sein, dass diese im Bereich der Resonanzfrequenz der zu behandelnden Tumorzellen liegt. Somit kann eine spezifische Anregung dieser Zellen erzielt werden.

Der HF-Ultraschallgenerator kann mit der Halteeinrichtung derart verbunden sein, dass dieser höhen- und/oder längsverstellbar und/oder seitenverstellbar und/oder schwenkbar ist. Die Positionierbarkeit kann also in wenigstens einer Achsrichtung, insbesondere in 2 oder in 3 Achsrichtungen (Raumkoordinaten) und/oder durch Verschwenken erfolgen. Somit kann der HF-Ultraschallgenerator gegenüber den zu behandelnden Tumorzellen vorteilhaft positioniert werden. Die Einstellbarkeit der Ausrichtung des HF-Ultraschallgenerators gegenüber der Halteeinrichtung ist besonders vorteilhaft, wenn die Halteeinrichtung fest am zu behandelnden Körper angeordnet ist. Die optimale Positionierung des HF-Ultraschallgenerators gegenüber den zu behandelnden Tumorzellen erfolgt dann durch ein Abstimmen der Position und Ausrichtung des HF-Ultraschallgenerators in der Halteeinrichtung.

Die Vorrichtung zur Zerstörung von Tumorzellen kann eine Ultraschalldiagnoseeinheit umfassen, die einen Diagnoseultraschallgenerator und eine Auswerteinheit zur Lokalisation bestimmter Tumorzellen umfasst. Somit kann eine einfache Lokalisation der Tumorzellen gewährleistet werden. Die Lokalisation kann mit der Positionierung des HF- und/oder NF-Ultraschallgenerators korreliert werden. Besonders bei einer exakten Positionierung des HF-Ultraschallgenerators kann der Behandlungserfolg weiter erhöht werden.

Die Steuerung kann mit einer Auswerteinheit derart gekoppelt sein, dass der HF-Ultraschallgenerator derart steuerbar ist, dass der Fokus der hochfrequenten Schwingungen auf die lokalisierten Tumorzellen einstellbar ist.

Die oben genannte Aufgabe wird des Weiteren durch eine Vorrichtung zur Zerstörung von Tumorzellen mit einem Hochfrequenz-Ultraschallgenerator und einer Steuerung gelöst, wobei die Steuerung derart angepasst ist, dass eine von dem HF-Ultraschallgenerator erzeugte, hochfrequente Schwingung derart gepulst oder pulsbar ist, dass die hochfrequente Schwingung durch eine niederfrequente Schwingung überlagert ist. Somit kann anstelle des HF- und des NF-Ultraschallgenerator ein einziger HF-Ultraschallgenerator treten, der durch eine entsprechende Ansteuerung und/oder Ausbildung Schwingungen erzeugt, die entsprechend hochfrequent und niederfrequent sind.

Nachfolgend wird die Erfindung anhand einiger Ausführungsbeispiele beschrieben, die mittels schematischer Abbildungen näher erläutert werden. Hierbei zeigen:
- Fig. 1: eine Behandlungsglocke nach einem erfindungsgemäßen Ausführungsbeispiel im Querschnitt; und
- Fig. 2: eine Steuerungseinheit zum Betreiben der Behandlungsglocke gemäß Fig. 1;
- Fig. 3: einen Querschnitt durch eine Behandlungsglocke nach einem erfindungsgemäßen Ausführungsbeispiel, der senkrecht zur Längsachse der Behandlungsglocke angeordnet ist; und
- Fig. 4: einen Querschnitt durch die Biopsievorrichtung der Anordnung nach einem erfindungsgemäßen Ausführungsbeispiel.

In der nachfolgenden Beschreibung werden für gleiche und gleichwirkende Teile dieselben Bezugszeichen verwendet.

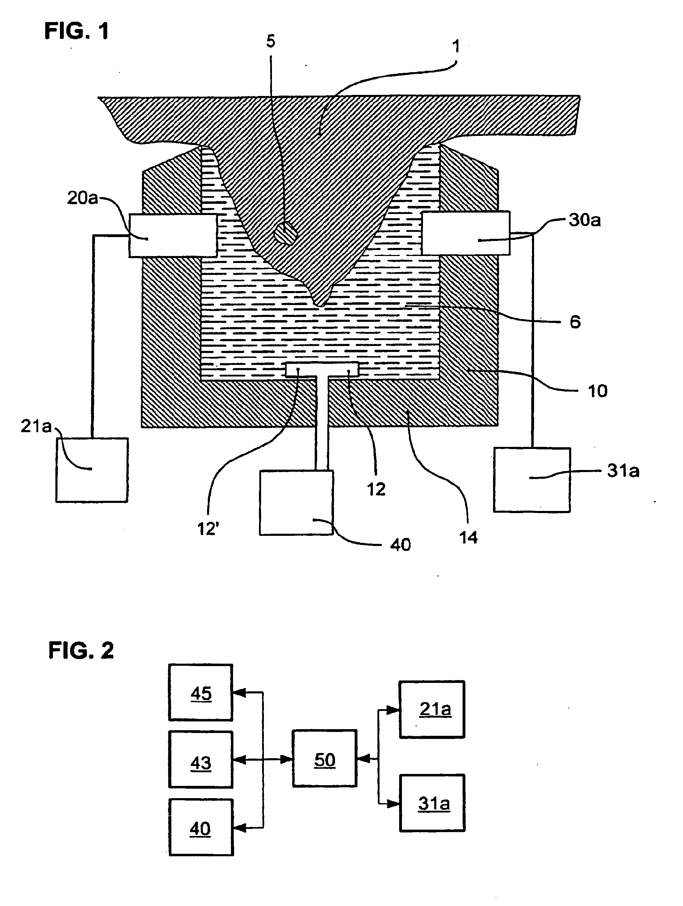

Die Fig. 1 zeigt eine Behandlungsglocke 10, die über ein zu behandelndes Gewebe 1, im vorliegenden Fall eine weiblich Brust, gestüipt ist. Die Behandiungsgiocke 10 ist zylindrisch ausgebildet und nach einer Seite hin offen. Die Öffnung liegt luftdicht an dem Gewebe 1 an. Im Innenbereich der Behandlungsglocke 10 bzw. im Behandlungsraum befindet sich ein Fluid 6 (z.B. Wasser). Zur Applikation von hochfrequenten und niederfrequenten Schwingungen umfasst die Behandlungsglocke 10 einen HF-Ultraschallkopf 20 und einen NF-Ultraschallkopf 30, die in einer Gegenüberstellung in den Seitenwänden der Behandlungsglocke 10 angeordnet sind.

Bei dem HF-Ultraschallkopf 20 und dem NF-Ultraschallkopf 30 handelt es sich um fokussierende Schallköpfe, die fokussierte Schallwellen in den Innenbereich der Behandlungsglocke 10 aussenden. Der HF-Ultraschallkopf 20 und der NF-Ultraschallkopf 30 sind derart ausgerichtet und werden derart angeregt, dass sich Schallfelder ergeben, deren maximale Leistung in einem fokussierten Zielbereich innerhalb des Behandlungsraums befindet. Der Zielbereich umfasst den Tumor 5.

Es ist ausreichend, wenn lediglich der schmalbandige HF-Ultraschallkopf 20 fokussierend ist. Es handelt sich hier also um einen hochintensiven fokussierten Ultraschall (HIFU). Der NF-Ultraschallkopf 30 muss nicht fokussierend sein, da das breitbandige Schallsignal, das dieser aussendet, derart gewählt ist, dass lediglich Tumorzellen und keine gesunden Zellen angeregt werden. Bezüglich des unterschiedlichen Anregungsverhaltens von Tumorzellen und gesunden Zellen wird auf die Einleitung verwiesen.

Um eine optimale Ausrichtung des HF-Ultraschallkopfes 20 und des NF-Ultraschallkopfes 30 zu gewährleisten, sind die entsprechenden Köpfe mechanisch beweglich an der Behandlungsglocke 10 angeordnet. Es ist möglich, die Höhe der Köpfe gegenüber dem Boden 14 der Behandlungsglocke 10 einzustellen. Des Weiteren lassen sich die Köpfe derart verschieben, dass sie weiter in den Innenraum bzw. Behandlungsbereich der Behandlungsglocke 10 hineinragen.

In einem weiteren Ausführungsbeispiel ist es denkbar, den HF-Ultraschallkopf 20 und den NF-Ultraschallkopf 30 derart frei zu lagern, dass diese beliebig ausgerichtet werden können.

Die Behandlungsglocke 10 umfasst einen Fluidzulauf 12 und einen Fluidablauf 12' am Boden 14. über den Fiuidzuiauf 12 kann das Fluid 6 in den Behandlungsbereich eingebracht werden, während über den Fluidablauf 12' Fluid 6 aus dem Behandlungsbereich abgeführt werden kann. Der Fluidzulauf 12 und der Fluidablauf 12' sind mit einer Unterdruckpumpe 40 verbunden, die unter anderem dazu ausgebildet ist, einen Unterdruck in dem Behandlungsbereich zu erzeugen, wodurch sich die Behandlungsglocke 10 an dem zu behandelnden Gewebe 1 festsaugt. Hierdurch kann eine effiziente Fixierung der Behandlungsglocke 10 gegenüber dem zu behandelnden Gewebe 1 erzielt werden. Eine andere Art der Fixierung, beispielsweise eine mechanische Fixierung (Anpressdruck) ist möglich.

Die Unterdruckpumpe 40 kann des Weiteren eine Entgasungseinrichtung 43 und eine Heiz-Kühl-Einheit 45 umfassen. Mittels der Unterdruckpumpe 40 wird das Fluid 6 aus dem Behandlungsbereich durch die Heiz-Kühl-Einheit 45 und die Entgasungseinrichtung 43 zirkuliert. Die Entgasungseinrichtung 43 filtert Gase aus dem Fluid 6 heraus. Durch das Verwenden eines entgasten Fluids 6 können Kavitationen in dem Fluid 6 vermieden werden. Die Entgasungseinrichtung 43 kann auch beim Auffüllen des Behandlungsbereichs mit Fluid 6 verwendet werden, um dort von Anfang an entgastes Fluid 6 bereitzustellen. Die Pumpe 40 ist auch als Überdruckpumpe geeignet. Durch eine Steuerung der Pumpe 40 und damit verbunden durch eine Druckänderung im Behandlungsraum ist das Kavitationsverhalten beeinflussbar.

Mit der Heiz-Kühl-Einheit 45 kann das Fluid 6 erwärmt oder abgekühlt werden. Durch die Erwärmung oder Abkühlung des Fluids 6 wird das Gewebe 1 mittelbar erwärmt. Durch ein Ändern der Gewebetemperatur kann eine vorteilhafte Behandlung erzielt werden, insbesondere kann die selektive Behandlung der Tumorzellen durch die niederfrequente Beschallung verbessert werden, da sich bei bestimmten Temperaturen das charakteristische Schwingungsverhalten von gesunden Zellen zugunsten der selektiven Anregung verändert.

Die Vorrichtung umfasst des Weiteren eine Steuerung 50 (Fig. 2), die mit der Unter-/Überdruckpumpe 40, der Entgasungseinrichtung 43, der Heiz-Kühl-Einheit 45, einem HF-Generator 21 und einem NF-Generator 31 verbunden ist. Die Steuerungseinheit 50 kann bei der Behandlung des Tumors 5 verschiedene Regel- und Steueraufgaben wahrnehmen. Insbesondere stellt sie den HF-Generator 21 und den NF-Generator 31 derart ein, dass die mit diesen verbundenen Ultraschallköpfen, nämlich der HF-Ultraschallkopf 20 und der NF-Ultraschallkopf 30, eine geeignete niederfrequente bzw. hochfrequente Schwingung erzeugen. Des Weiteren kann die Steuerungseinheit 50 die Temperatur des Fluids 6 und den Unterdruck bzw. Überdruck im Behandlungsbereich regeln.

Die Begriffe "Hochfrequenz-Ultraschallgenerator" und "Niederfrequenz-Ultraschallgenerator" sind im Sinne einer Ultraschalleinheit zu verstehen, die einen Ultraschallkopf 20, 30 und einen Ultraschallgenerator 21, 31 umfasst, wobei der Ultraschallkopf 20, 30 mit der Behandlungseinheit bzw. der Halteeinrichtung 10 verbunden ist. Da die Ultraschallköpfe 20, 30 von den Ultraschallgeneratoren 21, 31 angeregt werden und Ultraschallschwingungen emittieren, können die Ultraschallköpfe 21, 31 auch als Schwingköpfe bzw. letztendlich auch als eine Art von Generatoren bezeichnet werden.

In einem weiteren Ausführungsbeispiel (nicht gezeigt) umfasst die Behandlungsglocke 10 eine Diagnoseeinrichtung, mittels derer die Position des Tumors 5 exakt bestimmt werden kann. Diese Diagnoseeinrichtung kann entsprechende Signale an die Steuerungseinheit 50 liefern. Die Erfassung der Position des Tumors 5 durch die Diagnoseeinrichtung kann ebenfalls über geeignete Ultraschallsignale erfolgen. Entsprechende Detektionsverfahren sollten dem Fachmann bekannt sein.

In einem weiteren Ausführungsbeispiel kann die Steuerungseinheit 50 den HF-Ultraschallkopf 20 und/oder den NF-Ultraschallkopf 30 derart ansprechen, dass dieser bzw. diese als Diagnoseeinheit verwendet werden können. Es ist denkbar, den HF-Ultraschallkopf 20 und den NF-Ultraschallkopf automatisch gemäß der von der Detektionseinrichtung ermittelten Position auszurichten.

Für den Erfolg der Behandlung des Tumors 1 ist das Einstellen einer niederfrequenten Schwingung wichtig, die dazu geeignet ist, bei möglichst vielen Tumorzellen Resonanzphänomene auszulösen, die dann den Zelltod initiieren. Die hierfür geeignete Anregungsschwingung variiert ca. in einem Bereich von 10 bis 40 kHz. Da die geeignete Anregungsschwingung tumorspezifisch ist, kann durch eine Vorabbestimmung der Optimalfrequenz oder des Optimalfrequenzbereichs ein wesentlich besserer Behandlungserfolg erzielt werden. Hierfür wird Tumorgewebe entnommen und in einer Frequenzbestimmungseinheit analysiert. Diese Frequenzbestimmungseinheit umfasst einen monokristallinen Schwingkopf (Frequenzbereich 16 bis 80 kHz), der über einen geeigneten Generator angeregt wird. Dieser monokristalline Schwingkopf ist derart flächig ausgebildet, dass auf dessen Oberseite eine Vielzahl von Vertiefungen Platz finden. In diesen Vertiefungen kann die Gewebeprobe verteilt werden. Ein mit dem monokristallinen Schwingkopf verbundenes Oszilloskop bestimmt die Schwingungsfrequenz des monokristallinen Schwingkopfs bei einer Anregung durch den Generator. Eine Steuerung kann diese Schwingfrequenz so lange variieren, bis der Zelltod der Tumorzellen eintritt. Die so bestimmte Frequenz kann zur Behandlung in der Behandlungsglocke 10 verwendet werden. Es sind zahlreiche Verfahren denkbar, mit denen der Zelltod der Tumorzellen und die entsprechende Anregungsfrequenz bestimmt werden können. Vorteilhaft ist ein Betreiben der Frequenzbestimmungseinheit derart, dass der Zelltod visuell festgestellt werden kann. Beispielsweise kann das Zerplatzen der Tumorzellen wahrgenommen werden.

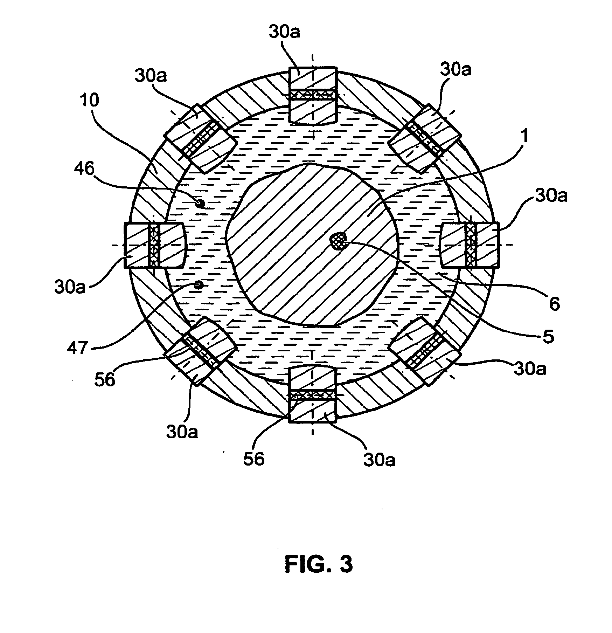

In Fig. 3 ist ein Querschnitt durch eine Behandlungsglocke 10 bzw. allgemein durch eine Behandlungseinrichtung zur Zerstörung von Tumorzellen dargestellt, wobei der Querschnitt senkrecht zur Mittelachse der Behandlungsglocke 10 verläuft (im Gegensatz zu dem Querschnitt gemäß Fig. 1, der entlang der Mittelachse verläuft). Der Hochfrequenz-Ultraschallkopf 20 und der zugehörige Hochfrequenz-Generator 21 sind außerhalb der Querschnittsebene angeordnet und daher in Fig. 3 nicht dargestellt. Die Behandlungsglocke gemäß Fig. 3 umfasst mehrere auf dem Umfang der Glocke angeordnete Niederfrequenz-Ultraschallköpfe 30a, insbesondere erste Niederfrequenz-Ultraschallköpfe 30a. Die Anordnung der ersten Niederfrequenz-Ultraschallköpfe 30a ist beispielhaft zu verstehen. Andere Positionen der einzelnen Köpfe 30a sind möglich. Bei dem Ausführungsbeispiel gemäß Fig. 3 sind acht Köpfe 30a vorgesehen. Eine andere Anzahl von Köpfen, beispielsweise in einem Bereich von zwei bis sechzehn ist möglich. Mehr als sechzehn Köpfe sind ebenfalls möglich. Jeder Kopf 30a weist ein Schwingelement 56 auf, beispielsweise ein Piezoelement, das von der (nicht dargestellten) Steuerung 50 bzw. Steuerungseinheit 50 angesteuert wird bzw. ansteuerbar ist. Die einzelnen ersten Niederfrequenz-Ultraschallköpfe 30a erzeugen jeweils eine unterschiedliche Frequenz. Es ist auch möglich, dass die einzelnen ersten Niederfrequenz-Ultraschallgeneratoren 30a, 31a jeweils in einem unterschiedlichen Frequenzband arbeiten. Durch Ansteuerung eines bestimmten Niederfrequenz-Ultraschallkopfes 30a kann der Tumor 5 mit der zur Behandlung vorgesehenen speziellen Frequenz beaufschlagt werden.

In der Behandlungsglocke 10 ist ein Behandlungsmedium 6 angeordnet, das die Übertragung der Schwingung von den einzelnen Köpfen 30a auf den Tumor 5 verbessert. Das Fluid 6 kann temperiert sein, insbesondere gekühlt, um eine möglichst große Temperaturdifferenz zwischen dem gesunden Brustgewebe 1 und dem durch den (nicht dargestellten) Hochfrequenz-Ultraschallgenerator 20, 21 lokal erwärmten Tumor einzustellen.

Dazu weist die Behandlungsglocke 10 eine (nicht dargestellte) Temperiereinheit, insbesondere Kühleinheit auf.

Ferner sind Temperatursensoren 46 und Drucksensoren 47 vorgesehen, die die Temperatur und den Druck des Fluids 6 messen.

Im Übrigen sind die im Zusammenhang mit dem Ausführungsbeispiel gemäß Figuren 1 und 2 beschriebenen Merkmale auch bei der Behandlungsglocke gemäß Fig. 3 vorgesehen.

Die einzelnen ersten Niederfrequenz-Ultraschallköpfe 30a haben den Vorteil, dass eine sehr schnelle Auswahl der geeigneten Schwingungsfrequenz ohne aufwendige Einstellung zur Behandlung des Tumors 5 möglich ist. Es ist auch möglich, die einzelnen Niederfrequenz-Ultraschallköpfe 30a durch einen oder mehrere breitbandig arbeitende Niederfrequenz-Ultraschallköpfe zu ersetzen, die ebenfalls die Auswahl bzw. Ansteuerung einer tumorspezifischen Schwingungsfrequenz ermöglichen.

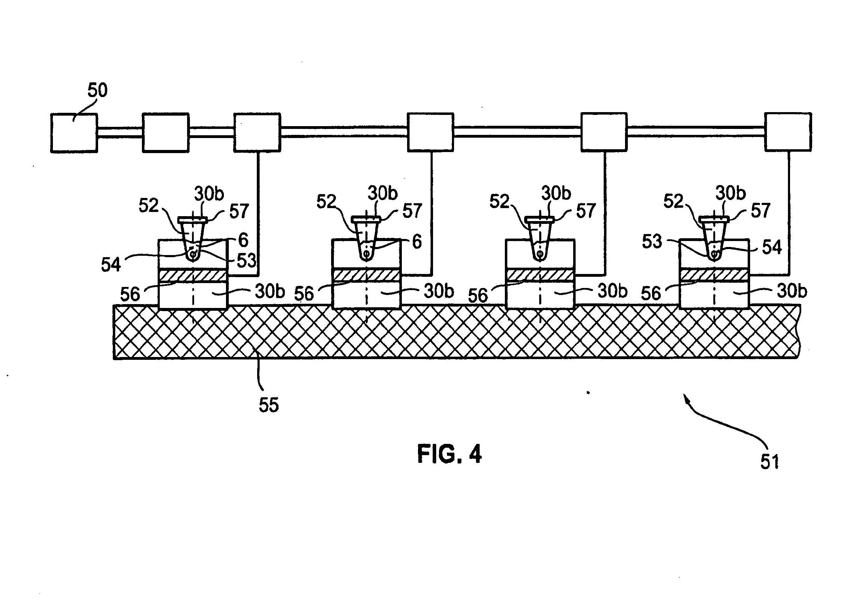

Die Vorrichtung gemäß Fig. 3 wird sowohl unabhängig von als auch zusammen mit einer Anordnung zur Zerstörung von Tumorzellen offenbart und beansprucht die ferner eine Biopsievorrichtung umfasst, die in Fig. 4 dargestellt ist. Die Biopsievorrichtung gemäß Fig. 4 ist ähnlich aufgebaut, wie die Behandlungsglocke 10 gemäß Fig. 3 insofern, als die Biopsievorrichtung mehrere zweite Niederfrequenz-Ultraschallschwingköpfe 30b aufweist, wobei durch jeweils einen ersten und zweiten Niederfrequenz-Ultraschallgenerator bzw. Schwingkopf 30a, 30b Schwingungen mit derselben Frequenz erzeugbar sind. Dies hat den Vorteil, dass Gewebeproben des zu behandelnden Tumors vor Durchführung der Behandlung im Hinblick auf eine möglichst wirksame Schwingungsfrequenz getestet werden können. Die so gefundene wirksame Schwingungsfrequenz wird dann durch die entsprechenden ersten Niederfrequenz-Ultraschallgeneratoren 30a der Behandlungsglocke 10 angelegt.

Die Biopsievorrichtung 51 umfasst eine Dämmplatte 55, auf der mehrere zweite Niederfrequenz-Ultraschallköpfe 30b, insbesondere in derselben Anzahl wie die ersten Niederfrequenz-Ultraschallköpfe 30a angeordnet sind. In Fig. 4 sind vier der insgesamt acht Schwingköpfe 30b dargestellt, wobei die Erfindung nicht auf die Anzahl von acht Köpfen eingeschränkt ist. Jeder Schwingkopf 30b umfasst ein Schwingelement 56, insbesondere ein Piezoelement, das durch die Steuerung 50 angesteuert wird. Ferner ist mit jedem Schwingkopf 30b eine Einzelaufnahme 52 verbunden, in der eine physiologische Flüssigkeit 6 bzw. ein Fluid 6 angeordnet ist. Im Fluid 6 befindet sich im Gebrauch eine Gewebeprobe des zu behandelnden Tumors 5. Die Einzelaufnahme 52 ist durch einen Deckel 57 verschlossen. Ferner sind Sensoren, insbesondere ein Beschleunigungssensor 53 und ein Temperatursensor 54 an der Einzelaufnahme 52 angebracht.

Die Steuerungseinheit 50 kann sowohl mit den zweiten Niederfrequenz-Ultraschallköpfen 30b der Biopsievorrichtung 51 als auch mit den ersten Niederfrequenz-Ultraschallköpfen 30a verbunden sein. Dies hat den Vorteil, dass nach der Bestimmung einer optimalen bzw. wirksamen Frequenz die Steuerungseinheit vom betreffenden zweiten Niederfrequenz-Ultraschallschwingkopf 30b der Biopsievorrichtung auf den entsprechenden ersten Niederfrequenz-Ultraschallkopf 30a der Vorrichtung umgeschaltet werden kann, der dieselbe Frequenz wie der Testkopf 30b aufweist. Dies hat den Vorteil, dass ohne zeitliche Verzögerung nach Bestimmung der wirksamen Frequenz diese zur Behandlung eingesetzt werden kann. Da das Schwingungsverhalten der Tumorzellen sich zeitlich schnell ändert, insbesondere kurz vor der Metastasierung durch die Bildung versteifender Proteinfilamente, wird ein möglichst unverfälschtes Testergebnis der Behandlung des Tumors 5 zugrunde gelegt.

Anstelle der einzelnen Niederfrequenz-Ultraschallschwingköpfen 30b, die jeweils eine bestimmte Testfrequenz bzw. ein Testfrequenzband aufweisen, können ein oder mehrere breitbandige Niederfrequenz-Ultraschallgeneratoren verwendet werden. Die mittels der breitbandigen Testköpfe bestimmte wirksame Schwingungsfrequenz wird dann den Behandlungsköpfen der Behandlungsglocke 10 zugrunde gelegt.

### Bezugszeichenliste

- 1: Gewebe
- 5: Tumor
- 6: Fluid
- 10: Behandlungsglocke
- 12: Fluidzufluss
- 12': Fluidabfluss
- 13: Boden
- 20: HF-Ultraschallkopf
- 21: HF-Generator
- 30a, 30b: NF-Ultraschallkopf
- 31a, 31b: NF-Generator
- 40: Unterdruckpumpe
- 43: Entgasungseinrichtung
- 45: Heiz-Kühl-Einheit
- 46: Temperatursensor
- 47: Drucksensor
- 50: Steuerungseinheit
- 51: Biopsievorrichtung
- 52: Einzelaufnahme
- 53: Beschleunigungssensor
- 54: Temperatursensor
- 55: Dämmplatte
- 56: Schwingelement
- 57: Deckel

## Patentansprüche

1. Anordnung zur Zerstörung von Tumorzellen, umfassend eine therapeutische Behandlungs-Vorrichtung mit wenigstens einem Hochfrequenz- Ultraschallgenerator (20, 21) zur Erzeugung einer zur lokalen Tumorerwärmung thermisch wirksamen, hochfrequenten Schwingung mit wenigstens 0,25 MHz, mehreren ersten Niederfrequenz- Ultraschallgeneratoren (30b, 31b) zur Erzeugung einer therapeutisch wirksamen, niederfrequenten Schwingung im Bereich von 1 kHz bis 200 kHz, wobei die ersten Niederfrequenz-Ultraschallgeneratoren in der Lage sind, jeweils eine einstellbare tumorspezifische, an das Stadium des Tumors angepasste Frequenz zu erzeugen, und einer Steuerung (50), die mit dem Hochfrequenz-Ultraschallgenerator (20, 21) und den ersten Niederfrequenz-Ultraschallgeneratoren (30a, 31a) verbunden ist derart, dass die Tumorzellen mit einer hochfrequenten, auf die Tumorzellen thermisch wirkenden Schwingung und mit einer niederfrequenten Schwingung beaufschlagbar sind, und
eine Biopsievorrichtung mit mehreren Einzelaufnahmen für Gewebeproben und mehreren zweiten Niederfrequenz- Ultraschallgeneratoren (30b, 31b), die mit den Einzelaufnahmen zur Beaufschlagung der Gewebeproben mit einer Schwingung zur Bestimmung der tumorspezifischen, an das Stadium des Tumors angepassten Frequenz wirkverbunden sind,
wobei die Anordnung dazu vorgesehen ist, durch jeweils einen ersten und zweiten Niederfrequenz- Ultraschallgenerator (30a, 31a, 30b, 31b) Schwingungen mit derselben tumorspezifischen, an das Stadium des Tumors angepassten Frequenz zu erzeugen.

2. Anordnung nach Anspruch 1,
**dadurch gekennzeichnet, dass**
die Steuerung (50) mit den zweiten Niederfrequenz-Ultraschallgeneratoren (30b, 31b) verbunden ist.

3. Vorrichtung nach Anspruch 1 oder 2,
**dadurch gekennzeichnet, dass**
eine Halteeinrichtung (10) vorgesehen ist, in der der Hochfrequenz-Ultraschallgenerator und der Niederfrequenz-Ultraschallgenerator gehalten sind.

4. Vorrichtung nach Anspruch 3,
**dadurch gekennzeichnet, dass**
die Halteeinrichtung (10) glocken- oder schüsselartig ausgebildet ist und zumindest teilweise einen Behandlungsraum begrenzt.

5. Vorrichtung nach Anspruch 4,
**gekennzeichnet durch**
eine Fluidpumpe (40), die zur Zirkulation und/oder zum Einbringen und/oder zum Abführen eines Fluids über eine Fluidleitung (12, 12') mit dem Behandlungsraum verbunden ist.

6. Vorrichtung nach Anspruch 5,
**dadurch gekennzeichnet, dass**
die Fluidpumpe (40) eine Heiz- und/oder Kühleinheit (45) zur Einstellung einer vorbestimmten Temperatur im Behandlungsraum umfasst.

7. Vorrichtung nach wenigstens einem der Ansprüche 1 bis 6,
**dadurch gekennzeichnet, dass**
wenigstens der Hochfrequenz-Ultraschallgenerator (20, 21) eine Schalllinse umfasst, die die erzeugte hochfrequente Schwingung fokussiert.

8. Vorrichtung nach wenigstens einem der Ansprüche 3 bis 6,
**dadurch gekennzeichnet, dass**
der Hochfrequenz-Ultraschallgenerator (20, 21) mit der Halteeinrichtung (10) verbunden ist derart, dass der Hochfrequenz-Ultraschallgenerator (20, 21) höhen- und/oder längsverstellbar ist und/oder seitlich verstellbar und/oder schwenkbar ist.

9. Vorrichtung nach wenigstens einem der Ansprüche 1 bis 8,
**dadurch gekennzeichnet, dass**
eine Ultraschall-Diagnoseeinheit vorgesehen ist, die einen DiagnoseUltraschallgenerator und eine Auswerteeinheit zur Lokalisation bestimmter Tumorzellen umfasst.

## Claims

1. Arrangement for destroying tumour cells, comprising
a therapeutic treatment apparatus having at least one high-frequency ultrasound generator (20, 21) for producing a high-frequency oscillation of at least 0.25 MHz that is thermally active for local tumour heating, having a plurality of first low-frequency ultrasound generators (30b, 31 b) for producing a therapeutically active low-frequency oscillation in the range from 1 kHz to 200 kHz, the first low-frequency ultrasound generators being capable, in each case, of producing an adjustable tumour-specific frequency matched to the stage of the tumour, and having a controller (50) which is so connected to the high-frequency ultrasound generator (20, 21) and to the first low-frequency ultrasound generators (30a, 31a) that the tumour cells can be subjected to a high-frequency oscillation acting thermally on the tumour cells and to a low-frequency oscillation, and
a biopsy apparatus having a plurality of individual receptacles for tissue samples and a plurality of second low-frequency ultrasound generators (30b, 31 b), which are in operative connection with the individual receptacles for subjecting the tissue samples to an oscillation for determining the tumour-specific frequency matched to the stage of the tumour,
wherein the arrangement is so provided that oscillations of the same tumour-specific frequency matched to the stage of the tumour are produced in each case by a first and second low-frequency ultrasound generator (30a, 31 a, 30b, 31 b).

2. Arrangement according to claim 1,
**characterised in that**
the controller (50) is connected to the second low-frequency ultrasound generators (30b, 31 b).

3. Apparatus according to claim 1 or 2,
**characterised in that**
there is provided a holding device (10), in which the high-frequency ultrasound generator and the low-frequency ultrasound generator are held.

4. Apparatus according to claim 3,
**characterised in that**
the holding device (10) is of bell-like or bowl-like construction and bounds, at least in part, a treatment space.

5. Apparatus according to claim 4,
**characterised by**
a fluid pump (40), which is connected to the treatment space for circulating and/or introducing and/or discharging a fluid by way of a fluid line (12, 12').

6. Apparatus according to claim 5,
**characterised in that**
the fluid pump (40) comprises a heating and/or cooling unit (45) for setting a predetermined temperature in the treatment space.

7. Apparatus according to at least one of claims 1 to 6,
**characterised in that**
at least the high-frequency ultrasound generator (20, 21) comprises an acoustic lens, which focuses the high-frequency oscillation produced.

8. Apparatus according to at least one of claims 3 to 6,
**characterised in that**
the high-frequency ultrasound generator (20, 21) is so connected to the holding device (10) that the high-frequency ultrasound generator (20, 21) is adjustable height-wise and/or longitudinally and/or is laterally adjustable and/or pivotable.

9. Apparatus according to at least one of claims 1 to 8,
**characterised in that**
an ultrasound diagnostic unit is provided which comprises a diagnostic ultrasound generator and an evaluation unit for locating particular tumour cells.

## Revendications

1. Dispositif pour la destruction de cellules tumorales, comprenant
un équipement de traitement thérapeutique comportant au moins un générateur d'ultrasons à hautes fréquences (20, 21) pour générer une oscillation à hautes fréquences thermiquement efficace pour l'échauffement local de la tumeur avec au moins 0,25 MHz, plusieurs premiers générateurs d'ultrasons à basses fréquences (30b, 31 b) pour générer une oscillation à basses fréquences thérapeutiquement efficace dans une plage de 1 kHz à 200 kHz,
dans lequel les premiers générateurs d'ultrasons à basses fréquences sont capables de générer respectivement une fréquence ajustable spécifique de la tumeur, adaptée au stade de la tumeur, et une commande (50) qui est reliée au générateur d'ultrasons à hautes fréquences (20, 21) et aux premiers générateurs d'ultrasons à basses fréquences (30a, 31) de telle sorte que l'on puisse soumettre les cellules tumorales à une oscillation à hautes fréquences agissant thermiquement sur les cellules tumorales et à une oscillation à basses fréquences et
un dispositif de biopsie comportant plusieurs réceptacles individuels pour des échantillons de tissus et plusieurs deuxièmes générateurs d'ultrasons à basses fréquences (30b, 31 b) qui sont reliés fonctionnellement aux réceptacles individuels pour soumettre les échantillons de tissu à une oscillation pour la détermination de la fréquence spécifique de la tumeur, adaptée au stade de la tumeur,
le dispositif étant prévu pour générer des oscillations avec la même fréquence spécifique de la tumeur, adaptée au stade de la tumeur par respectivement un premier et un deuxième générateur d'ultrasons à basses fréquences (30a, 31 a, 30b, 31 b).

2. Dispositif selon la revendication 1,
**caractérisé en ce que**
la commande (50) est reliée aux deuxièmes générateurs d'ultrasons à basses fréquences (30b, 31 b).

3. Dispositif selon la revendication 1 ou 2,
**caractérisé en ce que**
un dispositif de retenue (10) est prévu, dans lequel sont retenus le générateur d'ultrasons à hautes fréquences et le générateur d'ultrasons à basses fréquences.

4. Dispositif selon la revendication 3,
**caractérisé en ce que**
le dispositif de retenue (10) est de type cloche ou clé et limite au moins partiellement un espace de traitement.

5. Dispositif selon la revendication 4,
**caractérisé par**
une pompe de fluide (40) qui est reliée à l'espace de traitement pour la circulation et/ou pour l'insertion et/ou pour l'évacuation d'un fluide par une conduite de fluide (12, 12').

6. Dispositif selon la revendication 5,
**caractérisé en ce que**
la pompe de fluide (40) comprend une unité de chauffage et/ou de refroidissement (45) pour l'ajustement d'une température prédéterminée dans l'espace de traitement.

7. Dispositif selon au moins l'une des revendications 1 à 6,
**caractérisé en ce que**
au moins le générateur d'ultrasons à hautes fréquences (20, 21) comprend une lentille acoustique qui focalise l'oscillation à hautes fréquences générée.

8. Dispositif selon au moins l'une des revendications 3 à 6,
**caractérisé en ce que**
le générateur d'ultrasons à hautes fréquences (20, 21) est relié au dispositif de retenue (10) de telle sorte que le générateur d'ultrasons à hautes fréquences (20, 21) puisse être déplacé en hauteur et/ou en longueur et/ou puisse être déplacé et/ou basculé latéralement.

9. Dispositif selon au moins l'une des revendications 1 à 8,
**caractérisé en ce que**
une unité de diagnostic à ultrasons est prévue, qui comprend un générateur d'ultrasons de diagnostic et une unité d'analyse pour la localisation de certaines cellules tumorales.
